# EUROPÄISCHE PATENTANMELDUNG

(11) **EP 0 940 679 A2**
(43) Veröffentlichungstag der Anmeldung: **08.09.1999**
(21) Anmeldenummer: 99103013.1
(22) Anmeldetag: 15.02.1999
(51) Int. Cl.: G01N 33/00, G01N 27/12, G08B 17/117

(54) **System zur Branderkennung und Betriebsverfahren und Sensor für insbesondere dieses System**

(30) Priorität: 02.03.1998 DE 19808663
(71) Anmelder: SIEMENS MATSUSHITA COMPONENTS GmbH & CO. KG, 81541 München (DE)
(72) Erfinder: Fleischer, Maximilian Dr., 85635 Höhenkirchen (DE); Frank, Joachim, 85521 Ottobrunn (DE); Meixner, Hans, 85540 Haar (DE); Bernhardt, Klaus, 83059 Kolbermoor (DE)
(74) Vertreter: Epping, Wilhelm, Dr.-Ing.

(57) **Zusammenfassung**

System zur Branderkennung mit einem vorzugsweise Ga₂O₃-Sensor (10) mit amorpher Isolatorschicht als Filterschicht (14) auf der Ga₂O₃-Dünnschicht (11). Optimale Selektivität für als Brandmerkmal zu erkennenden Wasserstoff bei einer Betriebstemperatur von etwa 700°C.

## Beschreibung

Die vorliegende Erfindung bezieht sich auf die selektive Detektion von Wasserstoff zum Zweck der insbesondere frühzeitigen Branderkennung in Gebäuden und geschlossenen Industrieanlagen. Sie bezieht sich auch auf einen neuen Sensor.

Eine frühzeitige Branderkennung in im wesentlichen abgeschlossenen Räumen stellt eine wesentlich Maßnahme zur Vermeidung von Schäden an Personen und Sachwerten dar. Es wird daher im Zuge neuer Entwicklungen von insbesondere neuartigen Sensoren nicht nur die bloße Anwendung derselben, sondern auch weitergehende automatisierte Melde-Technik diskutiert. Hierzu werden vielfältige Sensoren und Aktoren miteinander vernetzt vorgesehen und betrieben. Eine erfolgte Branderkennung löst infolge entsprechend ausgebildetem Sensorsystem dann neben einer Warnung auch weitere Aktionen wie Notruf bei der Feuerwehr, Schließen der Fenster bei Abwesenheit von Personen und Öffnen derselben bei Anwesenheit der Personen, Abschalten der sonstigen Gasversorgung und ggfs. eine Auslösung eines vorhandenen Feuerlöschsystems aus.

Bisher eingesetzte Brandmelder und Brandmeldesysteme basieren im wesentlichen auf den Prinzipien eines Ionisationsmelders mit einem kleinen radioaktiven Präparat in der Mitte eines Detektionsvolumens, eines Rauchmelders, in dem durch Rauch verursachte Trübung der Luft oder Streuung von Licht gemessen wird, eines Temperaturfühlers, der abnormal hohe Temperaturen detektiert und der Verwendung unselektiver Gassensoren bzw. deren Systeme.

Von besonderem Interesse ist aber die selektive Messung der Anwesenheit von Wasserstoff in der umgebenden Luft, da die vorgenannten Systeme nur in beschränktem Maße einen aufgetretenen Brand von sonstigen Alarm auslösenden Umständen unterscheiden können. Eine in hohem Maße organisierte Brandmeldung und daraus abgeleitet einsetzende Brandbekämpfung erfordert aber eine zuverlässige Entscheidung, daß tatsächlich ein Brand vorliegt.

Der Erfindung liegt somit die Aufgabe zugrunde, ein System und (dafür) einen neuen Sensor und ein Betriebsverfahren anzugeben, das stark selektiv nur auf Wasserstoff anspricht und frei von Fehlalarm-auslösenden Querempfindlichkeiten und dgl. ist.

Diese Aufgabe wird jeweils mit einem System und einem Sensor und einem Betriebsverfahren gelöst, wie mit den Merkmalen der (einzelnen) Patentansprüche angegeben ist.

Der Erfindung liegt die Erkenntnis zugrunde, daß (von extremen Sonderfällen abgesehen) Wasserstoff in der Umgebungsluft in vornehmlich dem Haushalt und dgl. nur im Brandfall auftritt. Wegen seines hohen Dampfdruckes und seiner extremen Brennbarkeit kommt Wasserstoff in der hier bevorzugt in Frage kommenden Praxis weder als Treibgas in Spraydosen noch als Brenngas und dgl. zur Verwendung. Auch bei Koch-, Brat- und Backvorgängen, der beabsichtigten Verbrennung fossiler Brennstoffe mit Luftüberschuß, tritt kein Wasserstoff auf. Dagegen entsteht jedoch z.B. im Haushalt Wasserstoff im Brandfall, insbesondere bei Schwelbrand, bei dem die Verbrennung unter Sauerstoffmangel abläuft. Wasserstoff ist daher ein als weitestgehend sicheres Leitgas für Branderkennung zugrundegelegt.

Die vorliegende Erfindung ist zum einen ein zusammengesetztes System mit selektivem Wasserstoff-Sensor, mit einer Ansteuer- und Auslese-Elektronik für den Sensor, mit einer Sensorsignalverarbeitung und mit einem Ausgabemodul. Weitere zusätzliche Systemelemente können wahlweise vorgesehen sein, wie dies noch weiter ausgeführt wird.

Als für das System notwendiger selektiver Wasserstoffsensor eignen sich solche Sensoren, die auf der Basis folgender Wirkungsprinzipien arbeiten: Elektrochemische Zellen, gassensitive Feldeffekt-Transistoren und solche auf der Basis halbleitender Metalloxide.

Die Sensoren mit Halbleiteroxiden sind für die Erfindung bevorzugt, da sie sowohl kostengünstig als auch langlebig zu betreiben sind. Von besonderem Interesse sind dabei auf der Basis von insbesondere Ga₂O₃- oder WO₃-Dünnschichten funktionierende Sensoren. Ein solcher Sensor hat als neuer erfindungsgemäßer Sensor eine 50 nm bis 1000 nm, vorzugsweise etwa (± 20%) 300 nm dicke Beschichtung aus amorphem Isolatormaterial, vorzugsweise Siliciumdioxid oder Aluminiumoxid, hier als Filterschicht, auf der sensitiven Halbleiter-Dünnschicht.

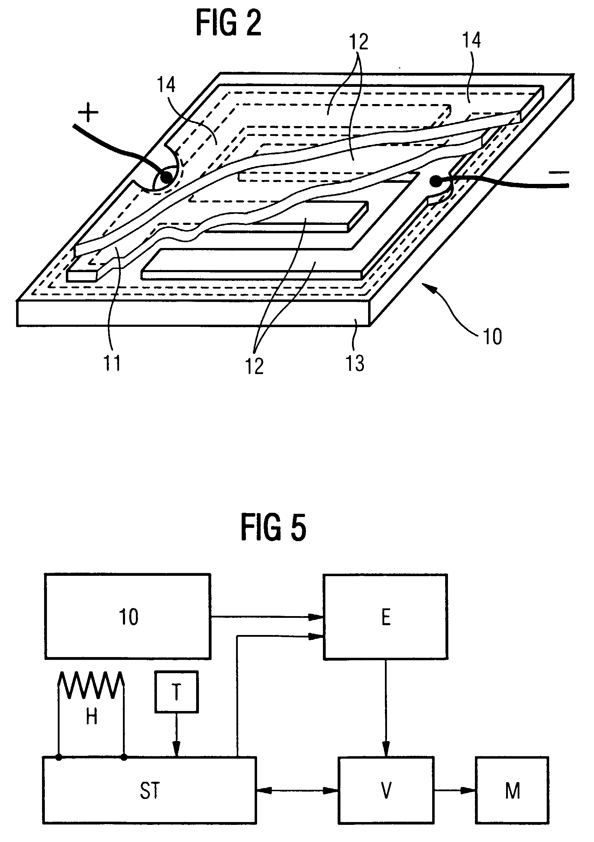

Einen solchen Sensor 10 zeigt die Figur 2. Mit 11 ist die Dünnschicht und mit 14 die Filterschicht auf der Dünnschicht (beide teilweise aufgebrochen dargestellt) bezeichnet. Die Elektrodenstruktur 12 liegt unterhalb der Dünnschicht 11 auf dem Substratplättchen 13. Auf der Unterseite des Substratplättchens ist eine bekannte Heizleiterstruktur (nicht dargestellt) vorgesehen. Die Abmessungen eines solchen Sensors sind derart, daß Temperaturänderungen desselben im Bereich unterhalb einer Minute vollziehbar sind.

Für die Erfindung hier ist bevorzugt ein solcher Ga₂O₃-Dünnschicht-Halbleitersensor zur Wasserstoff-Detektion verwendet. Seine Schichten 11 und 14 sind vorzugsweise aufgesputtert.
Figur 1 zeigt die Temperaturabhängigkeit der Selektivität der Sensitivität eines erfindungsgemäßen Ga₂O₃-Sensors für Wasserstoff.
Figur 2 zeigt einen solchen Sensor.
Figur 3 zeigt ein weiteres Diagramm.
Figur 4 zeigt die Abhängigkeit des elekrischen Widerstandes eines Sensors nach Figur 2 von der Wasserstoffkonzentration.
Figur 5 zeigt ein Blockschaltbild des Systems.

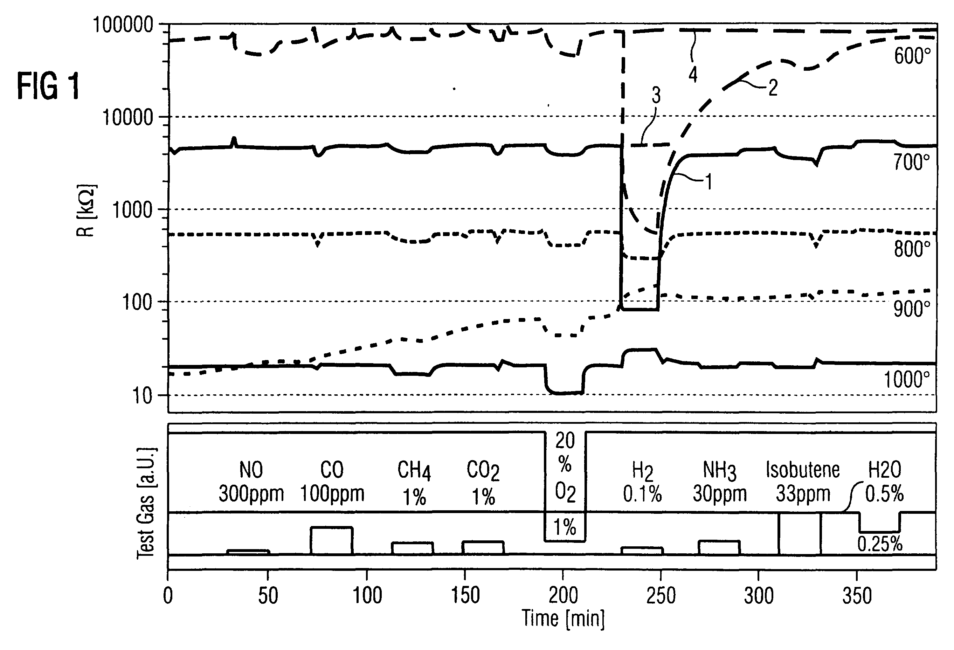

Die Figur 1 zeigt die Sensitivität S (wiedergegeben als elektrischer Widerstand) eines solchen Halbleitersensors 10 für Wasserstoff und sonstige wie in der Figur angegebene Gase/Stoffe an, und zwar für verschiedene Betriebstemperaturen zwischen 600°C und 1000°C. In dieser Figur ist auf der Ordinate der elektrische Widerstand einer solchen etwa 1 bis 3 µm dicken Ga₂O₃-Dünnschicht 11 mit darauf erfindungsgemäß 300 nm amorpher SiO₂-Schicht 14 aufgetragen, die auf der interdigitalen Elektrodenstruktur 12 auf dem Substrat 13 vorgesehen ist. Auf der Abszisse sind Zeitwerte in Minuten angegeben, nämlich für wie angegeben aus der Figur 1 entnehmbare Dauer erfolgte jeweilige Gasapplikation, und zwar in zeitlicher Folge aufeinander. Aus der Figur 1 ist die große selektive Empfindlichkeit/Sensitivität des Ga₂O₃-Sensors für Wasserstoff bei insbesondere 700°C und auch bei noch 600°C Temperatur des Sensors ersichtlich. Als jeweilige Bezugsgröße dieser Kurven gilt die Selektivität gegenüber der Applikation von Luft. Diesem Basiswert entsprechen die mit 3 und 4 bezeichneten, gestrichelt eingetragenen waagerechten Linien.

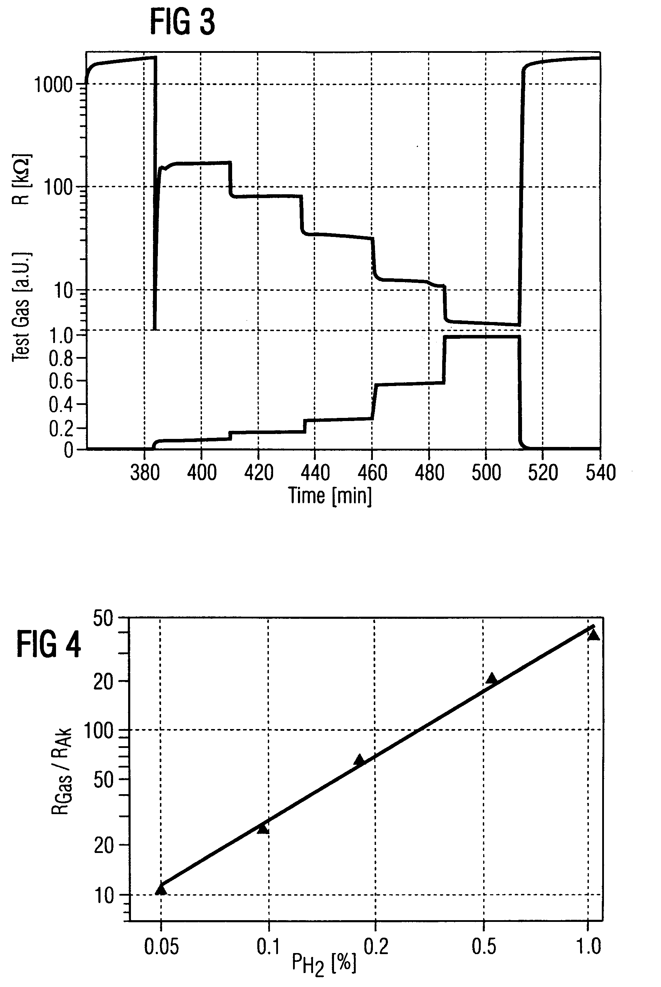

Eine für die Erfindung wichtige Sensoreigenschaft ist auch das in Figur 3 gezeigte Verhalten. Dort ist das zeitliche Ansprechen eines bei der Erfindung verwendeten Ga₂O₃-Dünnschicht-Sensors mit 300 nm amorpher Filterschicht aus SiO₂ nach Figur 1 für wechselnde Wasserstoff-Konzentrationen, und zwar bei einer Betriebstemperatur von 700°C, angegeben. Im unteren Teil der Figur 3 ist die von Zeit zu Zeit (siehe die Abszisse) gesteigerte Wasserstoff-Konzentration aufgetragen. Darüber ist der elektrische Widerstand R (wie in Figur 1) aufgetragen, der bei wie angegeben zeitlich stufenweise veränderter Wasserstoffkonzentration sich einstellt. Auffallend in Figur 3 ist die vorteilhaft rasche, nämlich im Sekundenbereich liegende, Anpassung des elektrischen Widerstandes der Ga₂O₃-Schicht an die jeweils vorgenommene Konzentrationsänderung.

Wie schon aus der Figur 1 ersichtlich, bedarf ein erfindungsgemäß eingesetzter Sensor einer Beheizung mit einer Heizleiterstruktur H auf/an dem Substratchip 13 des Sensors 10 und eine zugehörige Steuerung des zur Heizung zu liefernden Stromes J_{H}. Das Blockschaltbild der Figur 5 zeigt das Schema des für die Erfindung vorgesehenen Systems mit der zugehörigen Elektronik. Für das Messen der jeweils erreichten Sensortemperatur dient ein Temperaturmesser T bekannter Art. Gegebenenfalls kann die Temperatur auch aus dem temperaturabhängigen Widerstandsverhalten der Heizleiterstruktur abgeleitet werden. Es ist eine Elektronik E vorgesehen, die den elektrischen Widerstand der sensitiven Schicht des Sensors mißt (= jeweils auf der Ordinate aufgetragener Widerstand). Die Steuereinrichtung St ist mit dem Temperaturmesser T, der Heizung H, der Elektronik E und mit der Signalverarbeitung V und diese mit dem Ausgabemodul M verbunden.

Zur Sicherheit, daß nur im tatsächlichen Brandfalle ein Alarmsignal ausgelöst wird, ist die Sensor-Signalverarbeitung so ausgebildet, daß sie auch den zeitlichen Verlauf der während des Sensorbetriebs eingehenden Sensorsignale auswertet. Ein Brandalarm wird (nur) dann ausgelöst, wenn ein zeitlich starker Abfall des Sensorwiderstandes gemessen wird, nämlich der nur auf dem Auftreten von Wasserstoffgas, d.h. dem Entstehen/Vorliegen eines Brandes beruhen kann. Die Sensor-Signalverarbeitung führt also eine Plausibilitätsbetrachtung durch, in der mit der gemessenen Zeitkonstante der Widerstandsänderung des Sensors auf Brandentstehung 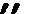 Ja" oder 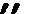 Nein" geschlossen wird. Mit derartigen Betrachtungen können insbesondere langsame Drifterscheinungen aufgrund einer Alterung und dgl. hinsichtlich der zu treffenden Aussage eliminiert werden. Diese Maßnahme zielt auf die Ermittlung der ersten Ableitung der gemessenen Sensorsignal-Werte nach der Zeit. Fakultativ kann die Signalverarbeitung auch zusätzlich noch derart ausgebildet sein, daß sie jeweils einen Selbsttest des Sensorelementes in entsprechend vorgegebenen Zeitabständen ausführt. Damit kann eine möglicherweise inzwischen eingetretene Fehlfunktion des Sensors aufgedeckt werden. Bei einem z.B. Ga₂O₃-Sensor besteht ein solcher Selbsttest z.B. in der Maßnahme des Abschaltens der schon erwähnten Heizung des Sensors und dem folgenden Nachprüfen, ob der Sensorwiderstand in Richtung des Wertes unendlich sich verändert hat. Es kann auch die Maßnahme eines zusätzlichen Aufheizens des Sensorchips vorgesehen sein, nämlich mit Überprüfung, ob der Sensorwiderstand die für ihn bekannte Widerstandsverringerung in korrektem Maße wiedergibt.

Zum System der Erfindung gehört auch ein Ausgabemodul für die überprüfte Weitermeldung des Vorliegens eines Brandes im Falle, daß der Sensor eine wie aus dem vorangehenden ersichtliche Widerstandsänderung gezeigt hat, bzw. eine solche an ihm gemessen worden ist.

Das erfindungsgemäße System kann auch noch zusätzlich fakultative Weiterbildungen einschließen. Eine solche ist z.B., nämlich um Heizenergie zu sparen und/oder die Lebensdauer des Sensors zu erhöhen, die Aufheizung des Sensors auf die vorgesehene Temperatur, z.B. 700 °C, nur intermittierend vorzunehmen. Dies ist insbesondere auch dann von Interesse, wenn lange Zuleitungen zum Sensor bedingt sind. Insbesondere kann dazu auch ein Energiespeichersystem am Ort des Sensors (ein Mini-Akkumulator, eine Kapazität oder eine Induktivität) vorgesehen sein, die fortlaufend aufgeladen wird und für den Zeitraum des Hochheizens des Sensorelements die akkumulierte elektrische Energie für das Aufheizen abgeben kann.

## Patentansprüche

1. System zur Branderkennung
mit einem Sensor (10), der selektiv auf Wasserstoffgas in der Raumluft anspricht,
mit einer Auslese-Elektronik (E) für die Meßwertsignale des Sensors (10), mit einer Ansteuerelektronik (St) für den Ablauf eines eingegebenen Betriebsprogramms,
mit einer Signalverarbeitung (V) und
mit einem Ausgabemodul (M).

2. Halbleitersensor, insbesondere in einem System nach Anspruch 1,
mit einem Substratchip (13), mit darauf vorgesehener Elektrodenstruktur (12), darauf befindlicher Halbleiter-Dünnschicht (11) und darauf aufgebrachter amorpher Isolatorschicht (14), diese mit einer Dicke zwischen 50 nm und 1000 nm,
sowie mit einer elektrischen Beheizung (H) mit Temperaturmesser (T),
wobei diese Beheizung für programmgeregelten Betrieb in dem System mit dessen Ansteuerelektronik (St) verbunden ist.

3. Sensor nach Anspruch 2,
bei dem eine Heizleiterstruktur rückseitig auf dem Substrat (13) des Halbleitersensors (10) als Beheizung und Temperaturmesser vorgesehen ist.

4. Sensor/System nach Anspruch 1, 2 oder 3,
wobei der Sensor (10) eine Ga₂O₃-Dünnschicht (11) mit einer Dicke zwischen 50 nm und 1000 nm aufweist.

5. Sensor/System nach Anspruch 1, 2 oder 3,
wobei der Sensor (10) eine WO₃-Dünnschicht (11) aufweist.

6. Sensor/System nach einem der Ansprüche 1 bis 5,
wobei der Sensor (10) eine 50 nm bis 1000 nm Dicke amorphe SiO₂-Schicht (14) als Isolatorschicht aufweist.

7. Sensor/System nach einem der Ansprüche 1 bis 5,
wobei der Sensor (10) eine 50 nm bis 1000 nm Dicke amorphe Al₂O₃-Schicht (14) als Isolatorschicht aufweist.

8. Sensor/System nach einem der Ansprüche 1 bis 7,
mit einer Isolatorschicht (14) mit einer Dicke zwischen 240 und 360 nm.

9. Sensor/System nach einem der Ansprüche 1 bis 8,
mit einer aufgesputterten Isolatorschicht (14).

10. Sensor/System nach einem der Ansprüche 1 bis 8,
mit einer nach einem CVD-(Chemical Vapor Deposition)-Verfahren aufgebrachten Isolatorschicht (14).

11. Verfahren zum Betrieb eines Systems nach einem der Ansprüche 1 bis 10,
bei dem die Entscheidung über das Auftreten eines Brandes vom Eintreten eines raschen Abfalls des elektrischen Sensorwiderstandes abhängig gemacht ist.

12. Verfahren nach Anspruch 11,
bei dem diese Entscheidung bei einer Temperatur von etwa 700°C des Ga₂O₃-Sensors getroffen wird.

13. Verfahren nach Anspruch 11 oder 12,
bei dem die Entscheidung anhand einer für den Sensor vorgebbaren Zeitkonstante der beobachteten Widerstandsänderung getroffen wird.

14. Verfahren nach Anspruch 11, 12 oder 13,
bei dem die Signalverarbeitung zeitlich beabstandete Selbsttests zum Erkennen von möglichen Fehlfunktionen ausführt.

15. Verfahren nach Anspruch 14,
bei dem ein Selbsttest mit zeitweisem Abschalten der Beheizung zwecks der Kontrolle, ob der Widerstand des Sensors im abgekühlten Zustand nach unendlich tendiert, ausgeführt wird.

16. Verfahren nach Anspruch 15,
bei dem während eines (zusätzlichen) Aufheizens des Sensors überprüft wird, ob der Sensorwiderstand die für ihn bekannte Widerstands-Verringerung wiedergibt.

17. Verfahren nach einem der Ansprüche 11 bis 16,
bei dem zur Einsparung von Heizenergie ein intermittierender Sensorbetrieb mit zwischenzeitlicher Absenkung der Temperatur des Sensors ausgeführt wird.

18. Verfahren nach Anspruch 17,
mit Absenkung auf eine Temperatur, bei der der Sensor (10) gerade noch auf Wasserstoff-Gas anspricht und
dann bei einem Auftreten eines Sensorsignals
mit folgendem Erhöhen der Temperatur auf die vorgesehene Betriebstemperatur mit optimaler Selektivität für Wasserstoff-Gas auszuführendes Überprüfen auf aktuelles Vorhandensein von H₂-Gas in der Raumluft.
